(19) Europäisches Patentamt
European Patent Office
Office européen des brevets

(11) Numéro de publication: **0 035 945**
**B1**

(12) # FASCICULE DE BREVET EUROPEEN

(45) Date de publication du fascicule du brevet: **21.12.83**

(21) Numéro de dépôt: **81400354.7**

(22) Date de dépôt: **06.03.81**

(51) Int. Cl.³: **C 07 C 129/12**, C 07 C 127/00, A 61 K 31/155, A 61 K 31/17

(54) **Nouvelles guanidines acylées, urées et urées substituées, leur procédé de fabrication et leur application en tant que médicaments.**

(30) Priorité: **07.03.80 FR 8005190**

(43) Date de publication de la demande:
**16.09.81 Bulletin 81/37**

(45) Mention de la délivrance du brevet:
**21.12.83 Bulletin 83/51**

(84) Etats contractants désignés:
**BE CH DE FR GB IT LI NL SE**

(56) Documents cités:
FR - A - 1 488 231
FR - A - 2 150 621
FR - A - 2 210 407
FR - A - 2 401 135
FR - M - 5 402
GB - A - 1 049 285
US - A - 3 437 691

HOUBEN-WEYL: METHODEN DER ORGANISCHEN CHEMIE, 4e édition, vol. VIII, Georg Thieme Verlag, STUTTGART (DE), pages 157-159 et 180-183

(73) Titulaire: **COMPAGNIE FRANCAISE DE SUCRERIE**
**336, rue Saint-Honoré**
**F-75001 Paris (FR)**

(72) Inventeur: **Anatol, Jésus**
**(décédé) (FR)**
Inventeur: **Berecoechea, Jean**
**1 Place Jean Moulin**
**F-51000 Reims (FR)**

(74) Mandataire: **Rinuy, Guy et al,**
**14, Avenue de la Grande Armée**
**F-75017 Paris (FR)**

(56) Documents cités:
JOURNAL OF POLYMER SCIENCE, vol. 7, partie A-1, no. 11, novembre 1969, Interscience Publishers, (US) Y. IWAKURA et al.: "Polythiazolines", pages 3075-3087

EP 0 035 945 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

# Nouvelles guanidines acylées, urées et urées substituées, leur procédé de fabrication et leur application en tant que médicaments

On connaît déjà un grand nombre de guanidines, d'urées et d'urées substituées pouvant être notamment utilisées dans le domaine de la thérapeutique.

On se référera entre autres au brevet français spécial de médicament n° 5.402 M; au brevet britannique n° 1.049.285; aux brevets français n° 2.150.621, 2.210.407 et 2.401.135; au brevet des Etats-Unis d'Amérique n° 3.437.691 ainsi qu'à l'article paru dans le Journal of Polymer Science, vol. 7, partie A—1, n° 11, novembre 1969, Interscience Publishers Y. Iwakura et al "Polythiazohnes" pages 3075—3087, pages 3078, 3085.

Or la présente invention a pour objet de nouvelles guanidines acylées, de nouvelles urées et urées substituées non révélées jusqu'à présent et répondant à la formule générale:

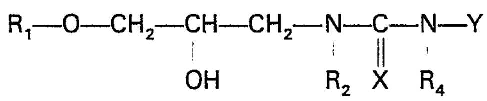

(I)

dans laquelle $R_1$ représente un cycle aromatique substitué ou non, $R_2$ représente un groupe cycloalkyle, alkyle, aryle ou arylalkyle, $R_4$ représente de l'hydrogène ou un groupe aryle diversement substitué, X est de l'oxygène ou un groupe imine: NH, et Y représente un groupe alkyle, aryle, alkyloxy ou aryloxy ou un atome d'hydrogène quand X représente de l'oxygène, ou le groupement $COR_3$ avec $R_3$ représentant un groupe aromatique, un groupe aryloxy, aralkyloxy ou alkyloxy quand X représente le groupe NH.

L'invention concerne également un procédé de fabrication de ces composés nouveaux.

Ce procédé consiste à faire réagir un oxyaminoalcool de formule:

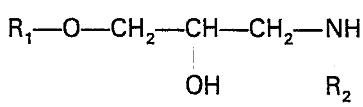

(II)

où $R_1$ et $R_2$ ont la signification ci-dessus avec un composé de formule:

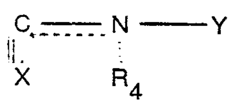

(III)

(où X représente O ou N et Y représente un groupe alkyle, aryle, alkyloxy ou aryloxy ou un atome d'hydrogène quand X représente de l'oxygène, ou le groupement $COR_3$ avec $R_3$ représentant un groupe aromatique, un groupe aryloxy, aralkyloxy ou alkyloxy quand X représente N), $R_4$ a la signification ci-dessus, par simple chauffage au reflux en présence d'un solvant approprié.

En procédant de la sorte, on peut obtenir, conformément à l'invention, des composés répondant à la formule générale (I) de la série des guanidines répondant de façon plus spécifique à la formule:

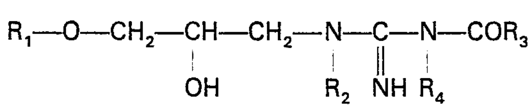

(Ia)

lorsque le composé de formule (III) est un cyanamide

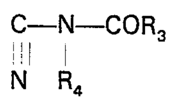

suivant l'équation:

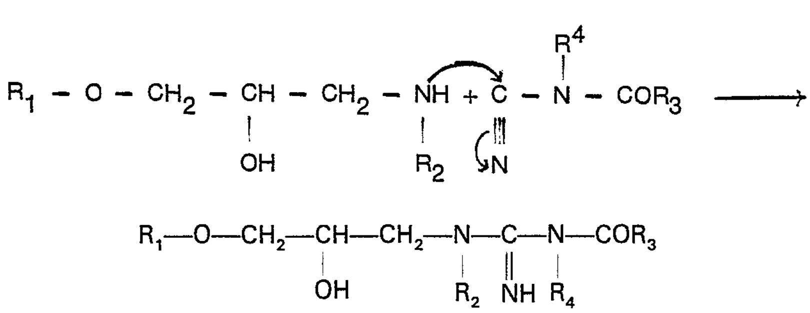

ou des composés répondant à la formule générale (I) de la série des urées et urées substituées répondant de façon plus spécifique à la formule:

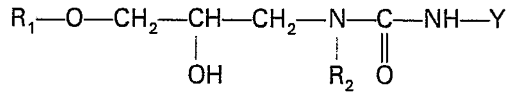

(Ib)

lorsque le composé de formule (III) est un isocyanate

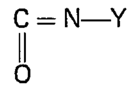

suivant l'équation:

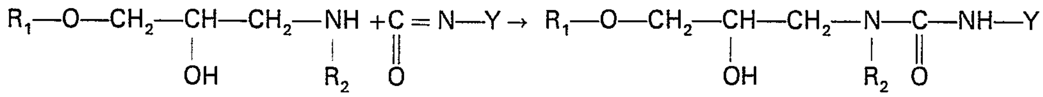

ou encore, dans le cas où Y représente de l'hydrogène, des composés:

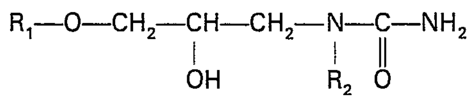

(Ic)

Les exemples suivants sont donnés à titre illustratif et nullement limitatif de l'invention.

Exemple 1

*(Méthyl-3 phényloxy-1, hydroxy-2-propyl)-1, cyclohexyl-1 benzoyl-3 guanidine — Composé SC3 300*

Dans un ballon surmonté d'un réfrigérant, on dissout 13,5 g (0,05 mole) de (méthyl-3 phényloxy-1 hydroxy-2 propyl)-cyclohexylamine dans 50 ml d'éthanol. On ajoute 7,2 g (0,05 mole) de benzoylcyanamide dissous dans 20 ml d'éthanol. On chauffe à reflux pendant 16 heures. On évapore l'éthanol sous pression réduite. On fait recristalliser l'huile résiduelle dans un mélange d'acétate d'éthyle et d'oxyde d'isopropyle. On obtient un produit fondant à 105°C.

*Analyse:* $C_{24}H_{31}O_3N_3$; P.M. = 409,51

| | *C* | *H* | *N* |
|---|---|---|---|
| Calculé: | 70,39 | 7,63 | 10,26 |
| Trouvé: | 70,58 | 7,83 | 9,90 |

Le tableau I ci-dessous donne les résultats obtenus en appliquant le même procédé, les produits répondant tous à la formule figurant en tête de ce tableau.

TABLEAU I

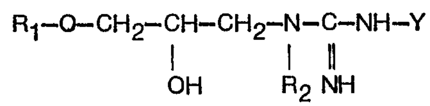

| Référence produit SCS | $R_1$ | $R_2$ | Y | Solvant recristallisation | F° | ANALYSE | C | H | N |
|---|---|---|---|---|---|---|---|---|---|
| 149 | 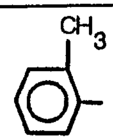 | $(CH_3)_2-CH-$ | 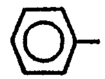 CO – | AcOEt + oxyde Isop. | 81 | Calculé :<br>Trouvé : | 68,26<br>68,09 | 7,36<br>7,39 | 11,31<br>11,10 |
| 150 | 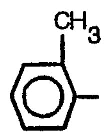 | $(CH_3)_2CH-$ | $C_2H_5-O-CO-$ | AcOEt | 105 | Calculé :<br>Trouvé : | 60,51<br>60,54 | 8,06<br>8,11 | 12,45<br>12,59 |
| 295 | 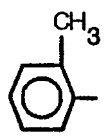 | $C_6H_{11}-$ | 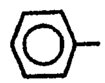 CO – | AcOEt + oxyde Isop. | 92 | Calculé :<br>Trouvé : | 70,38<br>70,37 | 7,63<br>7,66 | 10,26<br>10,03 |
| 151 | 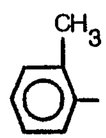 | $C_6H_{11}-$ | $C_2H_5-O(CO-$ | AcOEt + oxyde Isop. | 104 | Calculé :<br>Trouvé : | 63,63<br>63,53 | 8,28<br>8,35 | 11,13<br>11,10 |
| 296 | 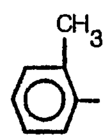 | 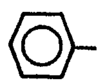 $CH_2-$ | $C_2H_5-O-CO-$ | AcOEt | 88 | Calculé :<br>Trouvé : | 65,43<br>65,48 | 7,06<br>7,16 | 10,90<br>10,93 |
| 297 | 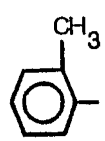 | $CH_3-(CH_2)_3-$ | $C_2H_5-O-CO-$ | AcOEt + oxyde Isop. | | Calculé :<br>Trouvé : | 61,52<br>61,65 | 8,32<br>8,42 | 11,96<br>11,93 |

TABLEAU I (bis)

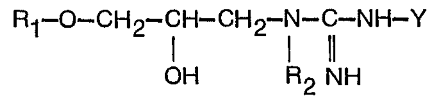

| Référence produit SCS | $R_1$ | $R_2$ | Y | Solvant recristallisation | F° | ANALYSE | C | H | N |
|---|---|---|---|---|---|---|---|---|---|
| 298 | 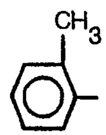 | $C_6H_{11}$– | 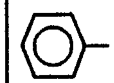 – $CH_2$–O–CO– | AcOEt ou EtOH + $H_{2}O$ | 90 | Calculé :<br>Trouvé : | 68,31<br>68,12 | 7.57<br>7,50 | 9,56<br>9,63 |
| 299 | 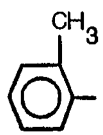 | H | 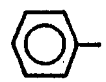 CO – | EtOH + $H_{2}O$ | | Calculé :<br>Trouvé : | 66,03<br>66,00 | 6,47<br>6,80 | 12,84<br>12,93 |
| 300 | 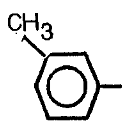 | $C_6H_{11}$ | 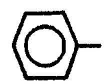 CO – | AcOEt + oxyde Isop. | 105 | Calculé :<br>Trouvé : | 70,39<br>70,58 | 7,63<br>7,83 | 10,16<br>9,90 |
| 152 | 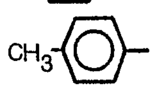 | $(CH_3)_2$–CH– | $C_2H_5$–O–CO– | AcOEt | 110 | Calculé :<br>Trouvé : | 60,51<br>60,61 | 8,07<br>8,12 | 12,45<br>12,48 |
| 146 | 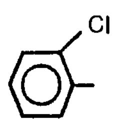 | $C_6H_{11}$– | $C_2H_5$–O–CO– | AcOEt + Ether Isop. | 100 | Calculé :<br>Trouvé : | 57,34<br>57,45 | 7,09<br>7,15 | 10,56<br>10,46 |
| 145 | 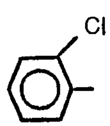 | $C_6H_{11}$– | 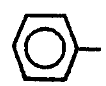 CO – | AcOEt + Hexane | 114 | Calculé :<br>Trouvé : | 64,24<br>64,24 | 6,56<br>6,59 | 9,77<br>9,85 |

TABLEAU I (bis)

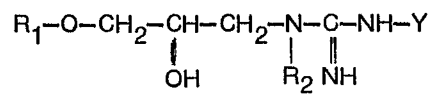

| Référence produit SCS | $R_1$ | $R_2$ | Y | Solvant recristallisation | F° | ANALYSE | | | |
|---|---|---|---|---|---|---|---|---|---|
| | | | | | | | C | H | N |
| 148 | 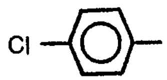 | $(CH_3)_2CH-$ | $C_2H_5-O-CO-$ | AcOEt + oxyde Isop. | 86 | Calculé :<br>Trouvé : | 53,69<br>53,75 | 6,76<br>6,80 | 11,74<br>11,68 |
| 301 | 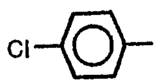 | $C_6H_{11}$ | 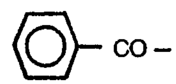 | AcOEt + Hexane | 118 | Calculé :<br>Trouvé : | 64,24<br>63,86 | 6,56<br>6,58 | 9,77<br>9,50 |
| 147 | 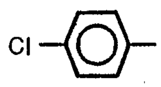 | $C_2H_{11}$ | $C_2H_5-O-CO-$ | EtOH + oxyde Isop. | 132 | Calculé :<br>Trouvé : | 57,34<br>57,41 | 7,09<br>7,05 | 10,56<br>10,52 |
| 144 | 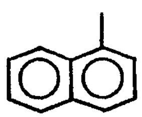 | $C_6H_{11}$ | 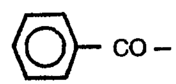 | AcOEt + Hexane | 120 | Calculé :<br>Trouve : | 72,78<br>72,68 | 7,01<br>6,95 | 9,43<br>9,48 |

Exemple 2

*(Methyl-2 phényloxy-1, hydroxy-2 propyl)-1 cyclohexyl-1 phényl-3 urée — Produit SC3 303*

On dissout 5,26 g (0,02 mole) de (méthyl-2 phényloxy-1 hydroxy-2 propyl)-cyclohexylamine dans 300 ml de benzène. On ajoute, 2,38 g (0,02 mole) de phénylisocyanate. On observe un léger échauffement ainsi que l'apparition d'un solide. On chauffe à reflux pendant 1 heure. Après refroidissement, on filtre et on fait recristalliser le produit dans l'éthanol. Point de fusion 164°C.

*Analyse:* $C_{23}H_{30}N_2O_3$; P.M. = 382,49

| | *C* | *H* | *N* |
|---|---|---|---|
| Calculé: | 72,22 | 7,90 | 7,32 |
| Trouvé: | 72,14 | 7,86 | 7,38 |

Le tableau II ci-après donne les résultats obtenus en appliquant le même procédé, les produits répondant tous à la formule figurant en tête de ce tableau.

TABLEAU II

$R_1-O-CH_2-CH-CH_2-N-CO-NH-Y$
OH R2

| Référence produit SCS | $R_1$ | $R_2$ | Y | Solvant recristallisation | F° | ANALYSE | C | H | N |
|---|---|---|---|---|---|---|---|---|---|
| 302 | 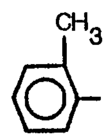 | $(CH_3)_2-CH$ | 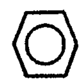 | EtOH + $H_{2}0$ | 165 | Calculé :<br>Trouvé : | 70,15<br>70,18 | 7,65<br>7,65 | 8,18<br>8,28 |
| 303 | 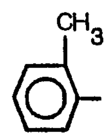 | $C_6H_{11}$ | 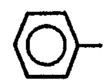 | EtOH | 164 | Calculé :<br>Trouvé : | 72,22<br>72,14 | 7,90<br>7,86 | 7,32<br>7,38 |
| 304 | 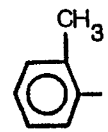 | $CH_3-(CH_2)_5-$ | 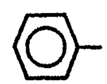 | EtOH + $H_{2}0$ | 120 | Calculé :<br>Trouvé : | 70,76<br>70,88 | 7,92<br>7,87 | 7,86<br>8,02 |
| 305 | 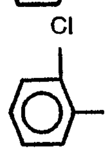 | $C_6H_{11}$ | 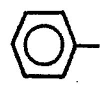 | EtOH | 170 | Calculé :<br>Trouvé : | 65,57<br>65,42 | 6,75<br>6,76 | 6,95<br>7,02 |
| 306 | 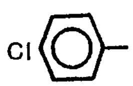 | $(CH_3)_2-CH-$ | 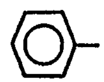 | EtOH | 152 | Calculé :<br>Trouvé : | 62,88<br>62,87 | 6,39<br>6,34 | 7,72<br>7,80 |

TABLEAU II (bis)

$$R_1-O-CH_2-\underset{OH}{\underset{|}{CH}}-CH_2-\underset{R_2}{\underset{|}{N}}-CO-NH-Y$$

| Référence produit SCS | $R_1$ | $R_2$ | Y | Solvant recristallisation | F° | ANALYSE | C | H | N |
|---|---|---|---|---|---|---|---|---|---|
| 126 | 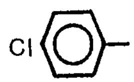 | $(CH_3)_2-CH-$ | $-(CH_2)_3-CH_3$ | AcOEt + Ether pétrole | 100 | Calculé :<br>Trouvé : | 59,50<br>59,40 | 7,88<br>7,89 | 7,80<br>8,08 |
| 307 | 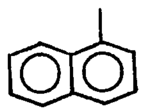 | $C_6H_{11}$ | 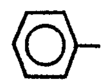 | EtOH + Ether pétrole | 150 | Calculé :<br>Trouvé : | 74,61<br>74,31 | 7,22<br>7,13 | 6,69<br>6,68 |
| 308 | 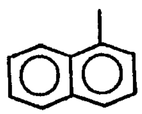 | $(CH_3)_2-CH-$ | 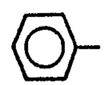 | AcOEt | 158 | Calculé :<br>Trouvé : | 72,99<br>72,95 | 6,92<br>6,84 | 7,40<br>7,45 |
| 127 | 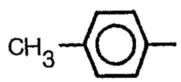 | $(CH_3)_2-CH-$ | $-(CH_2)_3-CH_3$ | AcOEt + Ether pétrole | 92 | Calculé :<br>Trouvé : | 67,04<br>67,05 | 9,38<br>9,37 | 8,69<br>8,78 |
| 128 | 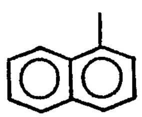 | $(CH_3)_2-CH-$ | $-(CH_2)_3-CH_3$ | AcOEt + Ether pétrole | 110 | Calculé :<br>Trouvé : | 70,36<br>70,28 | 8,44<br>8,44 | 7,81<br>7,87 |

Exemple 3

*(Chloro-2 phénoxy-1, hydroxy-2 propyl)-1 isopropyl-1 urée Produit SCS 313*

On dissout 2,8 g (0,01 mole) de chlorure de (chloro-2 phényloxy-1, hydroxy-2 propyl)-isopropyl-ammonium dans 20 ml d'eau et 5 ml d'éthanol. On ajoute 0,97 g (0,012 mole) de cyanate de potassium. On laisse reposer pendant 16 heures. On filtre et on fait recristalliser dans un mélange d'eau et d'éthanol. On obtient un produit fondant à 154°C.

*Analyse:* $C_{13}H_{19}ClN_2O_3$; P.M. = 286,80

| | *C* | *H* | *N* |
|---|---|---|---|
| Calculé: | 54,44 | 6,68 | 9,77 |
| Trouvé: | 54,41 | 6,70 | 9,73 |

Le tableau III ci-après donne les résultats obtenus en appliquant le même procédé, les produits répondant tous à la formule figurant en tête de ce tableau.

TABLEAU III

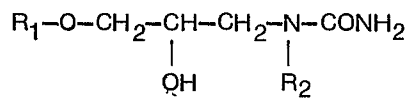

| Référence produit SCS | $R_1$ | $R_2$ | Solvant recristallisation | F° | ANALYSE | C | H | N |
|---|---|---|---|---|---|---|---|---|
| 309 | 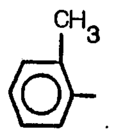 | $(CH_3)_2-CH-$ | EtOH + $H_{20}$ | 150 | Calculé :<br>Trouvé : | 63,13<br>63,08 | 8,33<br>8,34 | 10,52<br>10,50 |
| 310 | 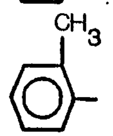 | $(CH_3)_3-C-$ | EtOH | 150 | Calculé :<br>Trouvé : | 64,26<br>64,19 | 8,63<br>8,49 | 9,99<br>9,96 |
| 311 | 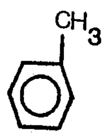 | $C_6H_{11}-$ | EtOH + $H_{20}$ | 170 | Calculé :<br>Trouvé : | 66,64<br>66,55 | 8,55<br>8,64 | 9,14<br>9,28 |
| 312 | 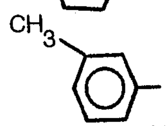 | $(CH_3)_2-CH-$ | EtOH + $H_{20}$ | 130 | Calculé :<br>Trouvé : | 63,13<br>63,26 | 8,33<br>8,43 | 10,52<br>10,42 |
| 313 | 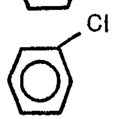 | $(CH_3)_2-CH-$ | EtOH + $H_{20}$ | 154 | Calculé :<br>Trouvé : | 54,44<br>54,51 | 6,68<br>6,70 | 9,77<br>9,73 |
| 314 | 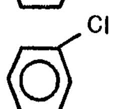 | $CH_3-(CH_2)_3-$ | EtOH + $H_{20}$ | 110 | Calculé :<br>Trouvé : | 55,89<br>55,84 | 7,04<br>6,95 | 9,31<br>9,05 |
| 315 | 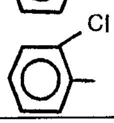 | $C_6H_{11}-$ | EtOH | 176 | Calculé :<br>Trouvé : | 58,79<br>58,80 | 7,09<br>7,02 | 8,57<br>8,47 |

TABLEAU III (bis)

$$R_1{-}O{-}CH_2{-}\underset{\displaystyle OH}{\underset{|}{CH}}{-}CH_2{-}\underset{\displaystyle R_2}{\underset{|}{N}}{-}CONH_2$$

| Référence produit SCS | $R_1$ | $R_2$ | Solvant recristallisation | F° | ANALYSE | C | H | N |
|---|---|---|---|---|---|---|---|---|
| 129 | 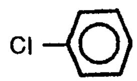 | $(CH_3)_2{-}CH{-}$ | EtOH | 146 | Calculé :<br>Trouvé : | 54,44<br>54,22 | 6,63<br>6,63 | 9,77<br>9,55 |
| 316 | 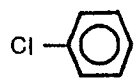 | $CH_3{-}(CH_2)_3{-}$ | AcOEt + oxyde Isop. | 90 | Calculé :<br>Trouvé : | 55,89<br>55,74 | 7,04<br>6,97 | 9,31<br>9,12 |
| 317 | 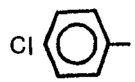 | $C_6H_{11}$ | EtOH | 158 | Calculé :<br>Trouvé : | 58,79<br>58,61 | 7,09<br>7,01 | 8,57<br>8,32 |
| 318 | 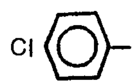 | 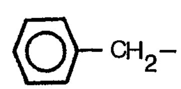 | EtOH + oxyde Isop. | 126 | Calculé :<br>Trouvé : | 60,97<br>60,92 | 5,72<br>5,63 | 8,37<br>8,20 |
| 131 | 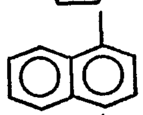 | $(CH_3)_2{-}CH{-}$ | EtOH | 152 | Calculé :<br>Trouvé : | 67,52<br>67,64 | 7,33<br>7,34 | 9,26<br>9,31 |
| 319 | 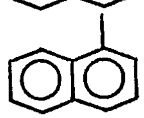 | $CH_3{-}(CH_2)_3{-}$ | EtOH + $H_20$ | 126 | Calculé :<br>Trouvé : | 68,33<br>68,41 | 7,65<br>7,54 | 8,85<br>8,73 |
| 320 | 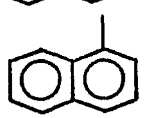 | $C_6H_{11}$ | EtOH + $H_20$ | 206 | Calculé :<br>Trouvé : | 70,15<br>70,09 | 7,65<br>7,73 | 8,18<br>8,23 |
| 130 | 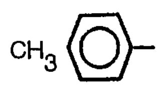 | $(CH_3)_2{-}CH{-}$ | EtOH + $H_20$ | 160 | Calculé :<br>Trouvé : | 63,13<br>62,96 | 8,33<br>8,46 | 10,52<br>10,59 |

Les composés de l'invention outre qu'ils présentent de l'intérêt dans les opérations de synthèses chimiques industrielles, trouvent des applications, suivant les molécules, dans divers domaines tels que ceux de l'agriculture, de l'industrie pharmaceutique, etc.

C'est ainsi notamment que l'on peut indiquer que les composés tels que SCS 149 et 150 ont des effets $\beta$-bloquants, que les composés tels que SCS 146, 147, 148, 151 ont des effets sympathomimétiques et que les composés SCS 128 et 131 ont des effets stimulants cardiaques les rendant intéressants dans le domaine thérapeutique.

Des essais effectués, en prenant comme élément de comparaison le Pindolol qui est, de tous les $\beta$-bloquants actuellement utilisés en thérapeutique, celui dont l'activité sympathomimétique intrinsèque est la plus importante, ont montré que:

— le composé SCS 146 possède une activité sympathomimétique importante;

— que le composé SCS 147 possède une telle activité sympathomimétique encore plus importante que celle du composé précédent;

— que le composé SCS 148 possède une certaine action $\beta$-bloquante masquée par des propriétés sympathomimétiques intrinsèques;

— que le composé SCS 149 possède d'indiscutables propriétés $\beta$-bloquantes (environ 60% des propriétés $\beta$-bloquantes du Pindolol) associées à des effets sympathomimétiques intrinsèques;

— que le composé SCS 150 possède des propriétés $\beta$-bloquantes adrénergiques (environ 70% des propriétés $\beta$-bloquantes du Pintolol) associées à des effets sympathomimétiques intrinsèques;

— que le composé SCS 151 présente les caractéristiques des sympathomimétiques;

— que le composé SCS 152 possède un certain effet $\beta$-bloquant.

Ces essais ont été effectués sur des chiens (de l'un et l'autre sexe) d'un poids compris entre 10 et 25 kg. Ces chiens sont anesthésiés par injection de Pentobarbital (0,1 ml/kg d'une solution à 6%) et de Chloralose (80 mg/kg). La pression artérielle est enregistrée au moyen d'un cathéter introduit dans l'artère tibiale et relié à une cellule de pression, cette pression étant inscrite sur un dynograph Beckman en même temps qu'un électrocardiogramme; ce dernier permet de contrôler le rythme cardiaque et la fréquence cardiaque, intégrée par un cardiotachymètre.

La pression artérielle et l'électrocardiogramme sont tout d'abord suivis pendant cinq minutes. Puis, une solution d'isoprénaline est perfusée dans une veine céphalique, à raison de 0,2 mcg/kg/min, soit 1 ml/min. Après cinq minutes de perfusion, la fréquence cardiaque a atteint un plateau de tachycardie. A ce moment, on branche une autre perfusion (dans la veine céphalique de l'autre patte) d'une solution de Pindolol ou du produit à étudier. Les composés de l'invention ont été solubilisés dans 5 ml de DMF. Le volume nécessaire à la perfusion est obtenu en complétant par une solution de DMF à 25% dans l'eau distillée. Tous les produits ont été injectés à raison de 25 mcg/kg/min.

On examine dans quelles mesures les produits sont susceptibles d'antagoniser la cardio-accélération d'une part et l'hypotension d'autre part provoquées par l'isoprénaline.

Au bout de 35 minutes de perfusion, on arrête l'injection d'isoprénaline. La perfusion du Pindolol ou du composé à étudier est interrompue au bout de 40 minutes. De cette façon, une dose totale de 750 mcg de produit est introduite dans le torrent circulaire durant la perfusion d'isoprénaline et il est possible, à l'arrêt de celle-ci, de mettre en évidence une éventuelle activité sympathomimétique.

Des essais sur bronches isolées et sur oreillettes isolées ont permis d'observer que le S.C.S. 149 présentait une activité $\beta$ 1 bloquante de type compétitif au niveau cardiaque et du type compétitif au niveau bronchique.

Quant à sa toxicité par la détermination de laquelle les expérimentations ont été réalisées sur deux espèces animales, à savoir le rat de souche OFA et la souris de souche OF1 par deux voies d'administration à savoir la voie orale et la voie intraveineuse, les résultats obtenus ont été les suivants:

Toxicité aigue du produit S.C.S. 149 adminstré par voie orale chez le rat

*Voie Orale*

La mort survient dans les 48 heures après le traitement.

| Espèce — Sexe | DL 50 (mg/kg) | Intervalle de Confiance à 95% |
|---|---|---|
| Rats mâles | 3332,7 | 2788,8 — 3876,6 |
| Rats femelles | 2932,6 | 2453,6 — 3411,5 |

Toxicité aigue de produit S.C.S. 149 administré par voie orale chez la souris

*Voie Orale*

La mort survient dans les 48 heures après le traitement.

| Espèce — Sexe | DL 50 (mg/kg) | Intervalle de Confiance à 95% |
|---|---|---|
| Souris mâles | 560,13 | 501,8 — 625,2 |
| Souris femelles | 604,81 | 539,6 — 669,9 |

Toxicité aigue du produit S.C.C. 149 administré par voie intraveineuse chez la souris

*Voie Intraveineuse*
DL 50 48 heures après le traitement.

| Espèce — Sexe | DL 50 (mg/kg) | Intervalle de Confiance à 95% |
|---|---|---|
| Souris mâles | 37,25 | 33,7 — 40,7 |
| Souris femelles | 38,81 | 35,8 — 42,0 |

Toxicité aigue du produit S.C.S. 149 administré par voie intraveineuse chez la rat

*Voie Intraveineuse*
La mort survient dans les 48 heures après l'administration du produit.

| Espèce — Sexe | DL 50 (mg/kg) | Intervalle de Confiance à 95% |
|---|---|---|
| Rats mâles | 35,31 | 33,5 — 37,1 |
| Rats femelles | 41,37 | 39,8 — 42,9 |

Toxicité aigue du produit S.C.S. 149 administré par voie intraveineuse chez la souris

*Voie Intraveineuse*
DL 50 14 jours après le traitement.

| Espèce — Sexe | DL 50 (mg/kg) | Intervalle de Confiance à 95% |
|---|---|---|
| Souris mâles | 37,25 | 33,7 — 40,7 |
| Souris femelles | 38,15 | 35,2 — 41,2 |

Le produit a été, pour l'administration orale, solubilisé dans la CMC à 2% dans l'eau; le volume administré était de 25 ml/kg chez la souris et chez le rat de 5 ml/kg pour la dose de 1 g/kg, 10 ml/kg pour 2 g/kg et 20 ml/kg pour 3,5 et 5 g/kg.

Le produit était administré par intubation oesophagienne.

Pour la voie intraveineuse, le produit était solubilisé dans:
— l'eau stérile chez la souris
— PEG 400 + eau stérile (1/3 + 2/3) chez le rat.

Le volume administré était:
— 1,25 ml/kg chez le rat
— 12,5 ml/kg chez la souris.

Le produit était injecté au niveau de la veine caudale.

Avant l'administration du produit, les animaux étaient privés de nourriture pendant 16 à 17 heures.

De même, des essais de toxicologie effectués sur 32 chiens des deux sexes et 160 rats, des deux sexes pendant six mois ont montré que le S.C.S. 149 pouvait être toléré par les rats jusqu'à la dose maximale journalière de 500 mg/kg et par les chiens jusqu'à la dose maximale journalière de 100 mg/kg.

Pour leur administration à l'homme, la posologie et la présentation du médicament seront celles actuellement connues pour les médicaments de la classe des propanolols utilisés pour le traitement de l'hypertension artérielle, des maladies ischémiques du coeur et des troubles du rythme de celui-ci. Ces

posologies sont, par exemple de 5 à 20 mg/jour, et la présentation celle de comprimés.

Des essais pharmacologiques ont montré aussi que:

— les composés SCS 128 et 131 constituaient à des doses appropriées relativement fortes (de l'ordre de 50 mg) des stimulants cardiaques.

Il va de soi que la présente invention a été décrite ici à titre purement explicatif et nullement limitatif et que toute modification utile pourra y être apportée sans sortir de son cadre tel que défini par les revendications ci-après.

## Revendications

1. Nouvelles guanidines acylées, urées et urées substituées répondant à la formule générale:

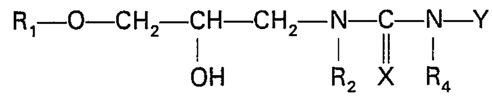

dans laquelle $R_1$ représente un cycle aromatique substitué ou non, $R_2$ représente un groupe cycloalkyle, alkyle, aryle ou arylalkyle, $R_4$ représente de l'hydrogène ou un groupe aryle diversement substitué, X est de l'oxygène ou un groupe imine: NH, et Y représente un groupe alkyle, aryle, alkyloxy ou aryloxy ou un atome d'hydrogène quand X représente de l'oxygène, ou le groupement $COR_3$ avec $R_3$ représentant un groupe aromatique, un groupe aryloxy, aralkyloxy ou alkyloxy quand X représente le groupe NH.

2. Guanidines selon la revendication 1, répondant à la formule:

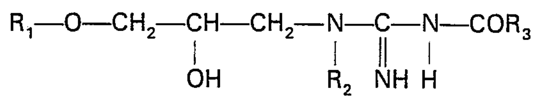

où $R_1$, $R_2$ ont la signification donnée à cette revendication 1 et $R_3$ représente un groupe aromatique, aryloxy, aralkyloxy ou alkyloxy.

3. Urées et urées substituées selon la revendication 1 répondant à la formule:

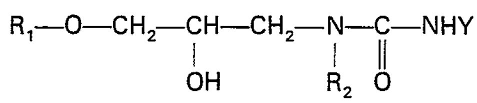

où $R_1$ et $R_2$ ont la signification donnée à cette revendication 1, et Y représente un groupe alkyle, aryle, alkyloxy, aryloxy ou un atome d'hydrogène.

4. Procédé de fabrication des composés selon la revendication 2, caractérisé en ce qu'il consiste à faire réagir un oxyaminoalcool de formule:

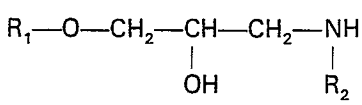

dans laquelle $R_1$ et $R_2$ ont la signification donnée à cette revendication avec un composé de formule:

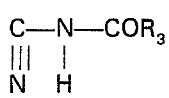

($R_3$ ayant la signification donnée à cette revendication), par simple chauffage au reflux, en présence d'un solvant approprié.

5. Composés selon l'une quelconque des revendications 1 à 3, en tant que médicaments ayant des effets $\beta$-bloquants pour le traitement de l'hypertension artérielle, des maladies ischémiques du coeur et des troubles du rythme de celui-ci.

6. Médicaments selon la revendication 5, caractérisés en ce qu'ils répondent à la formule générale:

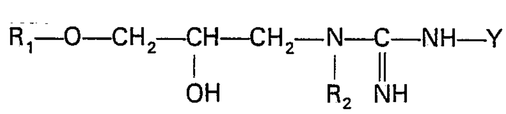

dans laquelle $R_1$ représente un groupe o-$ClC_6H_4$—, $R_2$ représente un radical $C_6H_{11}$— et Y un radical $C_2H_5$—O—CO—;

ou bien $R_1$ représente un groupe p-$ClC_6H_4$—, $R_2$ représente un radical $C_6H_{11}$— et Y un radical $C_2H_5$—O—CO—;

ou bien $R_1$ représente un groupe p-$ClC_6H_4$—, $R_2$ représente un radical $(CH_3)_2CH$— et Y un radical $C_2H_5$—O—CO—;

ou bien $R_1$ représente un groupe o-$CH_3C_6H_4$—, $R_2$ représente un radical $(CH_3)_2CH$— et Y un radical $C_6H_5$—CO—;

ou bien $R_1$ représente un groupe o-$CH_3C_6H_4$—, $R_2$ représente un radical $(CH_3)_2CH$— et Y un radical $C_2H_5$—O—CO—;

ou bien $R_1$ représente un groupe o-$CH_3$—$C_6H_4$—, $R_2$ représente un radical $C_6H_{11}$— et Y un radical $C_2H_5$—O—CO—

ou bien encore $R_1$ représente un groupe p-$CH_3C_6H_4$—, $R_2$ représente un radical $(CH_3)_2CH$— et Y un radical $C_2H_5$—O—CO—.

## Claims

1. Novel acylated guanidines, ureas and substituted ureas of the following general formula:

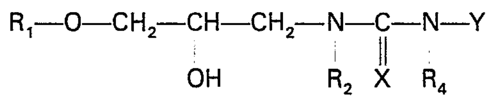

in which $R_1$ represents a substituted or unsubstituted aromatic cycle, $R_2$ represents a cycloalkyl, alkyl, aryl or arylalkyl group, $R_4$ represents hydrogen or a diversely substituted aryl group, X is oxygen or an imine group: NH, and Y represents an alkyl, aryl, alkyloxy or aryloxy group or a hydrogen atom when X represents oxygen, or the group $COR_3$, with $R_3$ representing an aromatic group, an aryloxy, aralkyloxy or alkyloxy group, when X represents the group NH.

2. Guanidines according to claim 1 of the following formula:

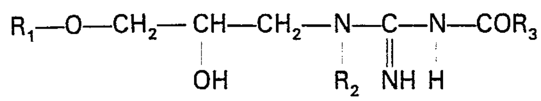

where $R_1$, $R_2$ have the same meaning as above in claim 1, and $R_3$ represents an aromatic, aryloxy, aralkyloxy or alkyloxy group.

3. Ureas and substituted ureas according to claim 1, of the following formula:

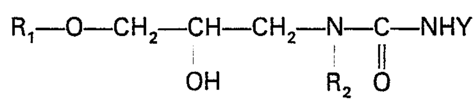

where $R_1$ and $R_2$ have the meaning given in claim 1, and Y represents an alkyl, aryl, alkyloxy, aryloxy group or a hydrogen atom.

4. A process for the preparation of compounds according to claim 2, characterized in that it consists of reacting an oxyaminoalcohol of the following formula:

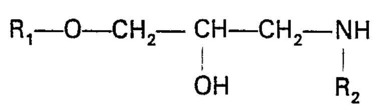

in which $R_1$ and $R_2$ have the meaning given in said claim, with a compound of the following formula:

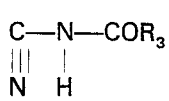

($R_3$ having the meaning given in said claim) through simple reflux heating, in the presence of a suitable solvent.

5. Compounds according to any one of claims 1 to 3, as medicaments having beta-blocking effects for treating arterial hypertension, ischemic sicknesses of the heart and rhythm disturbances of the latter.

6. Medicaments according to claim 5, characterized in that they are of the following general formula:

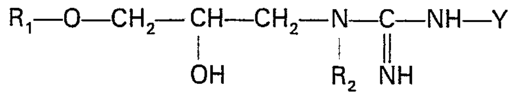

in which $R_1$ represents an o-$ClC_6H_4$— group, $R_2$ represents a $C_6H_{11}$— radical and Y a $C_2H_5$—O—CO— radical;

or $R_1$ represents a p-$ClC_6H_4$— group, $R_2$ represents a $C_6H_{11}$— radical, and Y a $C_2H_5$—O—CO— radical;

or $R_1$ represents a p-$ClC_6H_4$— group, $R_2$ represents a $(CH_3)_2CH$— radical and Y a $C_2H_5$—O—CO— radical;

or $R_1$ represents an o-$CH_3C_6H_4$— group, $R_2$ represents a $(CH_3)_2CH$— radical and Y a $C_6H_5$—CO— radical;

or $R_1$ represents an o-$CH_3C_6H_4$— group, $R_2$ represents a $(CH_3)_2CH$— radical and Y a $C_2H_5$—O—CO— radical;

or $R_1$ represents an o-$CH_3$—$C_6H_4$— radical, $R_2$ represents a $C_6H_{11}$— radical and Y a $C_2H_5$—O—CO— radical;

or else $R_1$ represents a p-$CH_3C_6H_4$— group, $R_2$ represents a $(CH_3)_2CH$— radical and Y a $C_2H_5$—O—CO— radical.

**Patentansprüche**

1. Acylierte Guanidine, Harnstoffe und substituierte Harnstoffe der allgemeinen Formel:

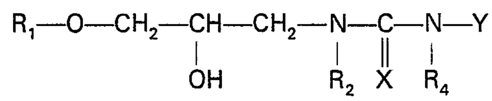

worin bedeuten: $R_1$ einen substituierten oder nichtsubstituierten, aromatischen Ring, $R_2$ eine Cycloalkylgruppe, Alkylgruppe, Arylgruppe oder Aralkylgruppe, $R_4$ ein Wasserstoffatom oder eine auf verschieden Art und Weise substituierte Arylgruppe, X ein Sauerstoffatom oder eine Imingruppe, =NH, und Y eine Alkylgruppe, Arylgruppe, Alkyloxygruppe oder Aryloxygruppe oder ein Wasserstoffatom, wenn X Sauerstoff bedeutet, oder die Gruppe $COR_3$, worin $R_3$ einen aromatischen Rest, eine Aryloxygruppe, Aralkyloxygruppe oder Alkyloxygruppe bedeutet, wenn X den Rest NH bedeutet.

2. Guanidine nach Anspruch 1 der Formel:

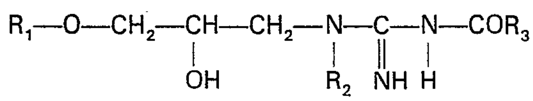

worin $R_1$ und $R_2$ die in Anspruch 1 angegebenen Bedeutung besitzen und $R_3$ einen aromatischen Rest, eine Aryloxygruppe, Aralkyloxygruppe oder Alkyloxygruppe bedeutet.

3. Harnstoff und substituierte Harnstoff nach Anspruch 1 der allgemeinen Formel:

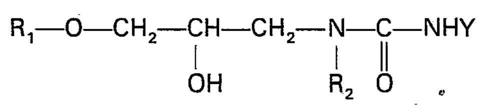

worin $R_1$ und $R_2$ die in Anspruch 1 angegebenen Bedeutung besitzen und Y eine Alkylgruppe, Arylgruppe, Alkyloxygruppe, Aryloxygruppe oder ein Wasserstoffatom ist.

4. Verfahren zur Herstellung von Verbindungen nach Anspruch 2, dadurch gekennzeichnet, daß ein Oxyaminoalkohol der Formel:

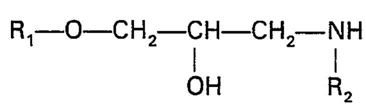

worin $R_1$ und $R_2$ die in Anspruch 2 angegeben Bedeutung besitzen, mit einer Verbindung der Formel:

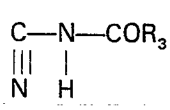

worin $R_3$ die in Anspruch 2 angegebene Bedeutung besitzt, durch einfaches Kochen unter Rückfluß in Anwesenheit eines geeigneten Lösungsmittels reagieren gelassen wird.

5. Verbindungen nach einem der Ansprüche 1 bis 3 als Arzneimittel mit β-blockierenden Effekten zur Behandlung von arterieller Hypertension, von ischämischen Herzkrankheiten und von Herzrhythmusbeschwerden.

6. Arzneimittel nach Anspruch 1, dadurch gekennzeichnet, daß sie der allgemeinen Formel entsprechen:

$$R_1{-}O{-}CH_2{-}\underset{\displaystyle OH}{\underset{|}{CH}}{-}CH_2{-}\underset{\displaystyle R_2}{\underset{|}{N}}{-}\underset{\displaystyle NH}{\underset{\|}{C}}{-}NH{-}Y$$

worin bedeuten:

$R_1$ die Gruppe o-$ClC_6H_4$—, $R_2$ den Rest $C_6H_{11}$— und Y den Rest $C_2H_5$—O—CO—; oder
$R_1$ den Rest p-$ClC_6H_4$—, $R_2$ den Rest $C_6H_{11}$— und Y den Rest $C_2H_5$—O—CO—; oder
$R_1$ den Rest p-$ClC_6H_4$—, $R_2$ den Rest $(CH_3)_2CH$— und Y den Rest $C_2H_5$—O—CO—, oder;
$R_1$ den Rest o-$CH_3C_6H_4$—, $R_2$ den Rest $(CH_3)_2CH$— und Y den Rest $C_6H_5$—CO—; oder
$R_1$ den Rest o-$CH_3C_6H_4$—, $R_2$ den Rest $(CH_3)_2CH$— und Y den Rest $C_2H_5$—O—CO—, oder
$R_1$ den Rest o-$CH_3$—$C_6H_4$—, $R_2$ den Rest $C_6H_{11}$— und Y den Rest $C_2H_5$—O—CO—; oder
$R_1$ den Rest p-$CH_3C_6H_4$—, $R_2$ den Rest $(CH_3)_2CH$— und Y den Rest $C_2H_5$—O—CO—.